# EUROPEAN PATENT APPLICATION

(11) **EP 2 980 714 A1**
(43) Date of publication of application: **03.02.2016**
(21) Application number: 15178066.5
(22) Date of filing: 23.07.2015
(51) Int. Cl.: G06F 19/00

(54) **APPARATUS AND METHOD OF PROVIDING NON-VISUAL INFORMATION AND COMPUTER-AIDED DIAGNOSIS SYSTEM USING THE SAME**

(30) Priority: 29.07.2014 KR 20140096751
(71) Applicant: Samsung Electronics Co., Ltd, Gyeonggi-do 443-742 (KR)
(72) Inventor: CHAE, Seung Chul, 114-201 Seoul (KR)
(74) Representative: Grünecker Patent- und Rechtsanwälte PartG mbB

(57) **Abstract**

Provided are a computer-aided diagnosis (CAD) system, a non-visual information providing apparatus and a method of providing non-visual information to facilitate making a diagnosis using a computer. The non-visual information providing apparatus includes a monitorer configured to detect condition information generated by a CAD apparatus, a non-visual information generator configured to generate non-visual information based on the detected condition information, and a non-visual information outputter configured to output the generated non-visual information to a user.

## Description

### CROSS-REFERENCE TO RELATED APPLICATION(S)

This application claims the benefit under 35 U.S.C. §119(a) of Korean Patent Application No. 10-2014-0096751, filed on July 29, 2014, in the Korean Intellectual Property Office, the entire disclosure of which is incorporated herein by reference for all purposes.

### BACKGROUND

### 1. Field

The following description relates to an apparatus and method of providing non-visual information to facilitate computer-aided diagnosis, and a computer-aided diagnosis system using the same.

### 2. Description of Related Art

Techniques for diagnosing patients by analyzing ultrasound images are recently being used in the field of medical imaging. Ultrasonography generally involves a doctor obtaining ultrasound images of a patient in real time by holding a probe in close contact with the body of the patient, thus capturing and determining a lesion or a suspected area while outputting ultrasound images onto a display screen. When inspecting suspicious area, the doctor may slowly move the probe over the area or let the probe linger there so as to observe the area of interest. In this case, once the ultrasound images are obtained by the probe, a computer-aided diagnosis (CAD) system may analyze the obtained images, detect a lesion, track a detected lesion, and provide the doctor with information regarding the detected lesion, such as whether the lesion includes benign or malignant tissues.

In a general CAD system, the analysis result of the images and the diagnosis may be processed, for example, by being colorized, having regions identified, or having information added thereto. The processed result may then be displayed by synthesizing the obtained information with an original image or overlaying it on a separate area of the original image. For example, a part that is to be displayed on a screen may be minimized in order not to interfere with a visual examination by a doctor. However, in a case where the diagnostic information is displayed on a screen, the doctor has to frequently move his/her eyes between the screen and the patient, and thus the increasing the likelihood of the doctor feeling tired or missing important information.

### SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

In one general aspect, a non-visual information providing apparatus includes a monitorer configured to detect condition information generated by a computer-aided diagnosis (CAD) apparatus, a non-visual information generator configured to generate non-visual information based on the detected condition information, and a non-visual information outputter configured to output the generated non-visual information to a user.

The non-visual information generator may be configured to select at least one expression of non-visual information including sound, voice or vibration, determine output duration and output intensity for the determined expression of non-visual information, and generate the non-visual information.

The condition information may include status information regarding a status of the CAD apparatus or event information regarding an event that occurred in the CAD apparatus.

The non-visual information generator may be configured to, in response to the detected condition information being event information, set the output duration to be equal to or less than a predetermined length of time and the output intensity to be equal to or greater than a predetermined intensity for the determined expression of non-visual information, and in response to the detected condition information being status information, set the output duration to be longer than the predetermined length of time and the output intensity to be weaker than the predetermined intensity of the event information.

The event information may include information generated due to an activation or deactivation of the CAD apparatus, a status change of the CAD apparatus, a detection of a lesion, a reception of an image, a speed of moving probe, an operational range of the probe, or a combination thereof.

The non-visual information generator may be configured to, in response to the detected condition information relating to detection of a lesion, determine whether the detected lesion has been previously detected, and in response to a determination that the lesion has been detected before, generate neither an event relating to the detected lesion nor non-visual information relating to a status.

The status information may include information generated due to a tracking of a lesion, a size of the lesion, a severity of a lesion, a probe position or a combination thereof.

In response to the detected condition information relating to a tracking of a lesion, the non-visual information generator may be configured to set the output duration and the output intensity in proportion to a time spent on tracking a lesion. In response to the detected condition information relating to a size or a severity of a lesion, the non-visual information generator may be configured to set the output duration and the output intensity in proportion to a size or severity of a lesion.

The non-visual information outputter may be configured to output the generated non-visual information to a user through a non-visual interface device. The non-visual interface device may be connected to the non-visual information providing apparatus via a wire or wireless communication. The non-visual interface device may include one or more of a headset, an earphone, a directional speaker, and a haptic device.

In another general aspect, a method of providing non-visual information involves detecting condition information generated in a computer-aided diagnosis (CAD) apparatus, generating non-visual information based on the detected condition information, and outputting the generated non-visual information to a user through a non-visual interface device.

The generating of the non-visual information may involve determining at least one expression of non-visual information from sound, voice, and vibration, based on the detected condition information; determining output duration and output intensity for the determined expression of non-visual information, based on the detected condition information; and generating the non-visual information using the determined output duration and output intensity.

The determining of the output duration and output intensity may involve: in response to the detected condition information being event information, setting the output duration to be equal to or less than a predetermined length of time and the output intensity to be equal to or greater than a predetermined intensity for the determined expression of non-visual information; and, in response to the detected condition information being status information, setting the output duration to be longer than the predetermined length of time and the output intensity to be weaker than the predetermined intensity.

In response to the detected condition information relating to tracking of lesion, the determining of the output duration and output intensity may involve setting the output duration and the output intensity in proportion to a time spent on tracking a lesion.

In response to the detected condition information relating to the size or severity of a lesion, the determining of the output duration and output intensity may involve setting the output duration and the output intensity in proportion to a size or severity of a lesion.

In another general aspect, a CAD system includes a CAD apparatus configured to generate events in association with operations performed by a probe and to change a status thereof, and a non-visual information providing apparatus configured to: monitor the CAD apparatus; in response to detection of an event or status change in the CAD apparatus, generate non-visual information based on the event or status change; and output the generated non-visual information to a user through a non-visual interface device.

The CAD apparatus may include a visual information generator configured to generate visual information based on operations performed by the probe, and a visual information outputter configured to output the generated visual information to a display screen.

The non-visual information providing apparatus may include a non-visual information generator configured to, in response to detecting an event, generate non-visual information to be output in a form of at least one of voice, sound or vibration for a duration equal to or less than a predetermined period of time at an output intensity equal to or greater than a predetermined intensity.

The non-visual information providing apparatus may include a non-visual information generator configured to, in response detecting a status change, generate non-visual information to be output in a form of at least one of voice, sound, or vibration for a duration less than the predetermined period of time at an output intensity less than the predetermined intensity.

The non-visual information providing apparatus may include a non-visual information outputter configured to output the generated non-visual information by controlling one or more non-visual interface devices, the one or more non-visual interfaces including at least one of a haptic device that is put on a user's body, an earphone, and a directional speaker.

In another general aspect, a method of providing non-visual information involves receiving, at a processor, information regarding a lesion detection, a lesion location or a lesion severity in a medical image obtained via a probe; generating non-visual information based on the received information; and outputting non-visual information via a non-visual interface device.

The outputting of the non-visual information may involve outputting a sound, voice, vibration or a combination thereof to indicate the lesion severity or a distance between the probe to a lesion by varying an intensity, duration or frequency of the non-visual information.

In another general aspect, there is provided a non-transitory computer readable storage medium storing instructions that cause a computer to perform the above described aspects of method.

In yet another general aspect, a non-visual information providing apparatus includes a processor configured to detect an event or a status change in a CAD apparatus and generate non-visual information based on the detected event or status change, and a non-visual interface device configured to output the generated non-visual information.

The non-visual interface device may include a headset, an earphone, a haptic device, a speaker, an amplifier, a vibrator, a magnet or a combination thereof, and the event or status change detected by the processor may indicate a lesion detection, a lesion severity or a lesion location within a medical image obtained by an imaging apparatus comprising an ultrasound probe, an MRI scanner, a PET scanner, an x-ray device or a combination thereof.

The processor may be configured to vary an intensity, duration or frequency of the non-visual information output via the non-visual interface device in proportion to: the lesion severity or a distance between a lesion to a probe of the imaging apparatus.

Other features and aspects will be apparent from the following detailed description, the drawings, and the claims.

### BRIEF DESCRIPTION OF THE DRAWINGS

FIG. 1 is a diagram illustrating an example of a computer-aided diagnosis (CAD) system.
FIG. 2 is a diagram illustrating an example of the CAD apparatus of FIG. 1.
FIG. 3 is a diagram illustrating a configuration of an operation controller according to the example of the CAD apparatus illustrated in FIG. 2.
FIG. 4 is a diagram illustrating a configuration of a diagnoser according to the example of the CAD apparatus illustrated in FIG. 2.
FIG. 5 is a diagram illustrating a configuration of a visual interface device according to the example of the CAD apparatus illustrated in FIG. 2.
FIG. 6 is a diagram illustrating an example of a non-visual information providing apparatus in accordance with the example of a CAD system illustrated in FIG. 1.
FIG. 7 is a diagram illustrating an example of a monitorer according to FIG. 6.
FIG. 8 is a diagram illustrating an example of a non-visual interface device according to FIG. 6.
FIG. 9 is a flowchart illustrating an example of a computer-aided diagnosis (CAD) method.
FIG. 10 is a flowchart illustrating an example of a CAD method performed by the CAD apparatus of FIG. 9.
FIG. 11 is a flowchart illustrating an example of a method of providing non-visual information performed by the non-visual information providing apparatus of FIG. 9.
FIG. 12 is a diagram illustrating an example of the application of a general CAD system.
FIGS. 13A and 13B are diagrams illustrating examples of the application of a CAD system in accordance with the exemplary embodiment.

Throughout the drawings and the detailed description, the same reference numerals refer to the same elements. The drawings may not be to scale, and the relative size, proportions, and depiction of elements in the drawings may be exaggerated for clarity, illustration, and convenience.

### DETAILED DESCRIPTION

The following detailed description is provided to assist the reader in gaining a comprehensive understanding of the methods, apparatuses, and/or systems described herein. However, various changes, modifications, and equivalents of the methods, apparatuses, and/or systems described herein will be apparent to one of ordinary skill in the art. The sequences of operations described herein are merely examples, and are not limited to those set forth herein, but may be changed as will be apparent to one of ordinary skill in the art, with the exception of operations necessarily occurring in a certain order. Also, descriptions of functions and constructions that are well known to one of ordinary skill in the art may be omitted for increased clarity and conciseness.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

Hereinafter, examples of an apparatus and method of providing non-visual information to facilitate computer-aided diagnosis and a computer-aided diagnosis system using the same will be described in detail with reference to the accompanying drawings. Herein, for convenience of description, the computer-aided diagnosis system will be referred to as a "CAD system"; the computer-aided diagnosis apparatus will be referred to as a "CAD apparatus"; and the apparatus that provides non-visual information to facilitate computer-aided diagnosis will be referred to as a "non-visual information providing apparatus."

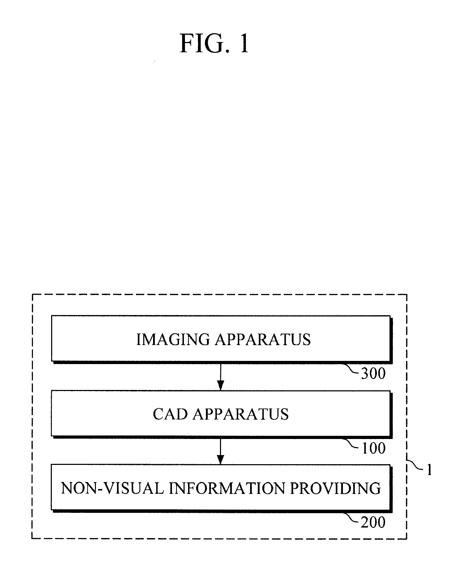

FIG. 1 illustrates an example of a CAD system.

Referring to FIG. 1, the CAD system 1 includes a CAD apparatus 100, a non-visual information providing apparatus 200, and an imaging apparatus 300.

In one example, the CAD apparatus 100, the non-visual information providing apparatus 200 and the imaging apparatus 300 may be implemented in a single hardware component. However, in another example, they may be implemented in separate hardware components that are connected to each other via wired/wireless communications. In yet another example, the non-visual information providing apparatus 200 may be mounted on or within a CAD apparatus 100 as a part thereof. In another example, the non-visual information providing apparatus 200 and the CAD apparatus 100 may be implemented in a single hardware component, and the imaging apparatus is detachably connected to the non-visual information providing apparatus 200 and the CAD apparatus 100.

The CAD apparatus 100 obtains medical images from the imaging apparatus 300, analyzes the obtained medical images to detect a lesion or a suspected area, track the lesion, and/or determine whether the lesion is benign or malignant. In addition, in response to obtaining medical images or making a diagnosis, the CAD apparatus 100 visibly outputs the medical images or the diagnosis to provide to a user. For example, the CAD apparatus 100 may visually display the medical images on a display. Further, the CAD apparatus 100 may generate an event or status change thereof in response to executions of various operations and diagnoses.

In one example, the CAD apparatus 100 may be one that obtains, using a probe of the imaging apparatus 300, real-time, ultrasound images of a patient; the CAD apparatus 100 may also analyze and diagnose the obtained ultrasound images. Hereinafter, the CAD apparatus 100 that diagnoses real-time ultrasound images is described as an example, for convenience of description. However, the description may employ a CAD apparatus that analyzes and diagnoses medical images obtained by various technologies such as CT, MRI, and the like.

The non-visual information providing apparatus 200 monitors the CAD apparatus 100 in that it oversees the latter's process of various operations and/or diagnoses status, thus allowing the former to keep track of any changes in condition, such as, the occurrence of an event or status changes. In addition, in response to detecting such condition information, the non-visual information providing apparatus 200 may generate non-visual information suitable for the detected condition information and output the generated non-visual information through a non-visual interface device to the user.

The non-visual information may be in the form of, for example, verbal or audio or haptic feedback. This haptic feedback may consist of vibration or other tactile sensations such as weight, pressure, texture, and the like. Furthermore, one or more expressions of the aforementioned non-visual information, such as, sound, voice, or haptic feedback, may be combined to generate different non-visual information suitable for different condition information. The non-visual interface device is a hardware device that output the non-visual information to senses of a user. The non-visual interface device may include a speaker, an earphone, an amplifier, a vibrator, a magnetic coil and/or other hardware components.

The imaging apparatus 300 may obtain medical images via ultrasonography, optical coherence tomography, CT, MRI, and other known medical imaging technologies. The imaging apparatus 300 may include a hand-held ultrasound probe, a hand-held optical coherence tomography (OCT) scanner, a scanner for CT or MRI, or other portable or stationary medical imaging device.

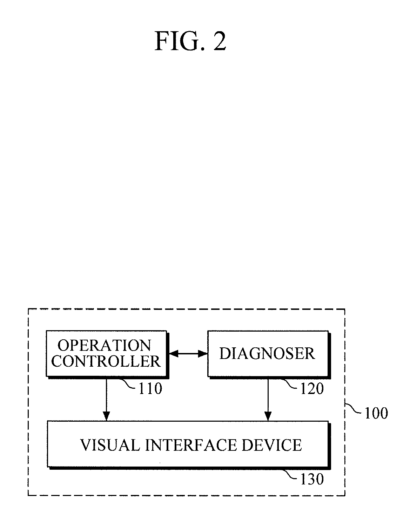

FIG. 2 illustrates an example of the CAD apparatus 100 of FIG. 1.

Referring to FIG. 2, the CAD apparatus 100 may include an operation controller 110, a diagnoser 120, and a visual interface device 130.

The operation controller 110 may control each module of the CAD apparatus 100, for example, the diagnoser 120, the visual interface device 130, and the other modules that are not illustrated herein, allowing them to perform their operations. The operation controller 110 and the diagnose 120 may include one or more processors, controllers and/or memories. For example, the operation controller 110 may activate and/or deactivate the CAD apparatus in response to a user's input. In addition, the operation controller 110 may activate a probe attached to the imaging apparatus to acquire an image, and control the diagnoser 120 to diagnose based on the acquired patient image. Then, once a diagnosis is made, the operation controller 110 may control the visual interface device 130 to output the diagnosis as visual information on a display. At this time, the operation controller 110 may cause a predetermined event to occur or update a status in accordance with an operation by each module.

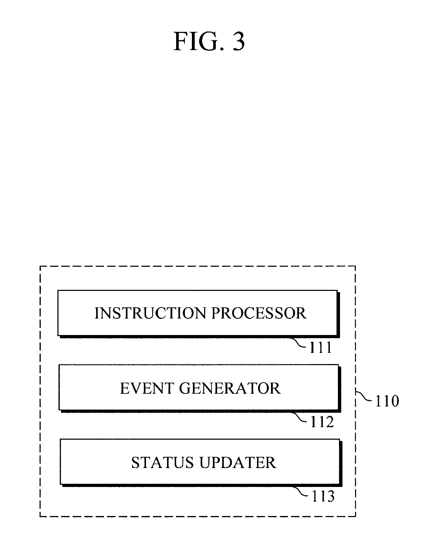

FIG. 3 illustrates an example of a configuration of the operation controller of FIG. 2. Referring to FIG. 3, the operation controller 110 may include an instruction processor 111, an event generator 112, and a status updater 113.

The instruction processor 111 creates various control instructions, and transmits the generated control instructions to corresponding modules in order to control the modules. For example, when the user acquires an image of an affected part of a patient by putting an ultrasound probe in close contact with the affected part, the instruction processor 111 may generate a control instruction to control the diagnoser 120 so that it may make its diagnosis by analyzing the acquired image. In addition, the instruction processor 111 may generate a necessary control instruction based on a user's input.

For example, in response to a user entering a start/end command to the CAD apparatus 100 by manipulating a start button or a switch equipped within the CAD apparatus 100, the instruction processor 111 may generate a control instruction based on the user's input and may activate or deactivate the CAD apparatus. Furthermore, in the event that the user finds a lesion or a suspected region in an image and enters a command using the probe to visually display the result, the instruction processor 111 may generate a control instruction to control the visual interface device 130 that displays the lesion or suspected region on the screen.

The event generator 112 generates events in response to operations executed based on the control of the instruction processor 111. The event generator 112 may generate an event in response to a sporadic operation among various operations of the CAD apparatus 100.

For example, in response to the CAD apparatus 100 starting or ending its operation, the event generator 112 may generate a start or end event. Also, in the event that the operational status of the apparatus is changed, the event generator 112 may generate an operational status change event; in the event that an initial image from the probe is received, an image reception event may be generated; in the event that a lesion is detected by analyzing the received image, the event generator 112 may generate a lesion detection event. In addition, in the event that the speed of a moving probe exceeds a predetermined range, the event generator 112 may generate a probe speed warning event, and in the event that the probe leaves the affected part of the patient, the event generator 112 may generate an abnormal input signal event.

The status updater 113 updates the status of the CAD apparatus 100 in the event that the operational status or diagnostic status continues for a longer period than the duration of an event.

For example, the status updater 113 may update the status of the CAD apparatus to an activation status in response to the CAD apparatus 100 being turned on and operating normally. Once the CAD apparatus 100 normally receives images from the probe at a normal and constant rate for a predetermined time period, the status updater 113 may update the status of the CAD apparatus to a normal input signal status. Further, in the event that a lesion is detected and the CAD apparatus 100 is tracking the detected lesion, the status updater 113 may update the status to a lesion tracking status. In addition, in response to determining a size or severity of the detected lesion based on the tracking of the lesion, the status updater 113 may update the status of the CAD apparatus 100 to a lesion size or severity determination status as a sub-status. Furthermore, in the event that the probe is removed from the affected part of the patient for more than a predetermined period of time, the status updater 113 may change the status of the CAD apparatus 100 to an abnormal input signal status.

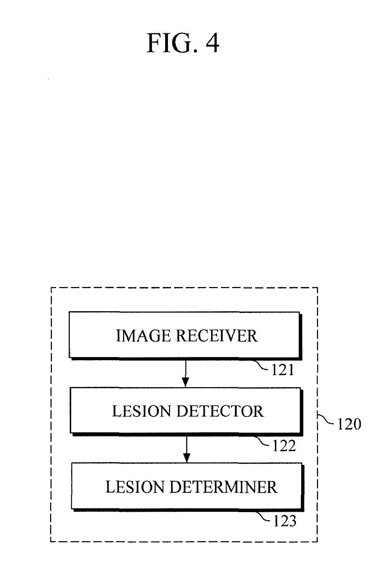

Referring back to FIG. 2, the diagnoser 120 receives images from the probe, detects and tracks a lesion by analyzing the received images, and makes a diagnosis by determining whether the detected lesion is malignant or benign. FIG. 4 illustrates an example of a configuration of the diagnoser 120 in detail.

Referring to FIG. 4, the diagnoser 120 includes an image receiver 121, a lesion detector 122, and a lesion determiner 123.

The image receiver 121 receives images that are acquired by placing the probe in close contact with a patient's body. The image receiver 121 may receive the images from the probe in real time on a frame-by-frame basis.

The lesion detector 122 detects a lesion or a suspected region by analyzing the received image, and tracks the lesion or suspected region in the sequentially input images. The lesion detector 122 may extract information regarding the features of a lesion that is necessary for lesion detection, and may then detect the lesion using the information. Information regarding the features of a lesion is a value obtained by extracting what features are present in a lesion, and this is done through an image data processing, whereby the features may indicate characteristics that define whether a lesion is benign or malignant. For example, the information regarding the features of a lesion may include computer-recognizable values, such as morphological characteristics (e.g. shape, margin, and boundary of a lesion as well as its textural characteristics).

The lesion determiner 123 is what determines whether the detected lesion is malignant or benign. At this time, the lesion determiner 123 may make a determination by applying a previously built diagnostic model to the extracted information regarding the features of the lesion.

Referring back to FIG. 2, the visual interface device 130 generates diagnostic information as visual information, and displays the visual information to the screen, wherein the diagnostic information contains information regarding the features of a detected lesion and extracted lesion as well as an assessment of whether the lesion is malignant or benign.

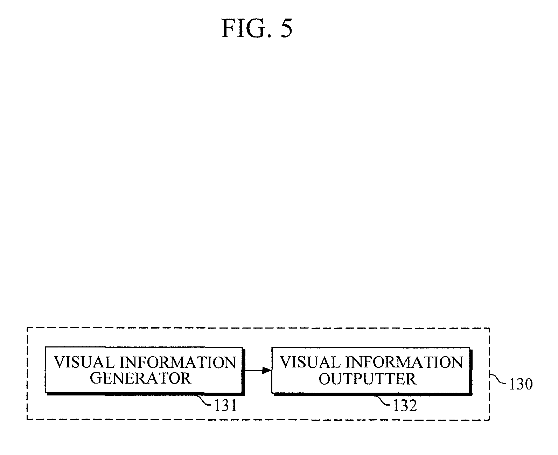

FIG. 5 illustrates an example of a configuration of the visual interface device shown in FIG. 2. Referring to FIG. 5, the visual interface device 130 includes a visual information generator 131 and a visual information outputter 132. The visual information generator 131 may include one or more processors and memories. The visual information outputter 132 may include one or more display, such as an LCD display or a touch screen.

Once the diagnostic information is generated, the visual information generator 131 may generate visual information by applying various predefined visual elements to each of diagnostic information such that the user can easily recognize the generated visual information.

For example, when a lesion is detected from the received image, the visual information generator 131 may create visual information by displaying an image onto the screen - an image wherein the visual information generator may place an identification mark that it has determined fit, for example, a cross, a rectangle, a circle, or the like, at a position in the lesion image. Furthermore, the visual information generator 131 may also determine a color, line thickness, or size of the identification mark. Alternately, evaluating based on information regarding the features of a lesion as well as its malignancy, the visual information generator 131 may determine various visual factors, such as a size or color of the lesion that is to be displayed, and then generate visual information based on the determinations. In this case, the visual factors that are applied to each piece of diagnostic information may be defined beforehand.

In response to receiving the image from the probe, the visual information outputter 132 may display the received image on the screen of a display, so that the visual information thus generated by the visual information generator 131 is displayed at a predefined position on the screen.

In order to indicate that the detected lesion is being tracked, the visual information outputter 131 may, at this time, continuously display the visual information at a corresponding position in each of the image frames that are consecutively output to the screen.

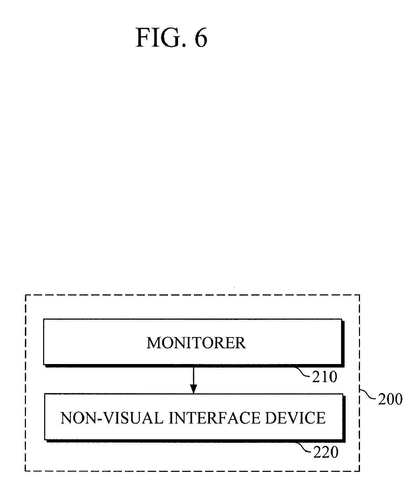

FIG. 6 is a diagram illustrating an example of a non-visual information providing apparatus in accordance with the example of CAD system illustrated in FIG. 1.

Referring to FIG. 6, the non-visual information providing apparatus 200 includes a monitorer 210 and a non-visual interface device 220. The monitorer 210 and the non-visual interface device 220 may include one or more processors and/or memories. The non-visual interface device 220 may also include a speaker, an amplifier, a vibrator, a magnetic coil, and/or other hardware feed-back generating devices.

The monitorer 210 receives information regarding operations of the CAD apparatus 100 and detects condition information of the CAD apparatus 100. Examples of condition information include occurrences of events or status changes of the CAD apparatus 100.

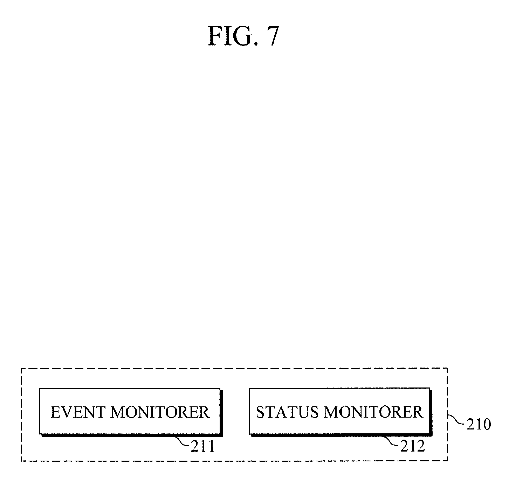

FIG. 7 illustrates in detail the monitorer of FIG. 6. Referring to the example illustrated in FIG. 7, the monitorer 210 includes an event monitorer 211 and a status monitorer 212.

The event monitorer 211 monitors a variety of events that occur in the CAD apparatus 100. Various events may be generated within the CAD apparatus 100 due to an activation or deactivation of the CAD apparatus 100, status changes of the CAD apparatus, a detection of a lesion in a medical image, a reception of an image from an imaging apparatus, a change in the speed of moving a probe, an operational range of the probe and the like.

The status monitorer 212 monitors the status of the CAD apparatus 100, and detects the status change of the CAD apparatus 100 that occurs in response to the operation of each module and/or diagnosis performed by the CAD apparatus 100.

Referring back to FIG. 6, in response to the monitorer 210 detecting the condition information, such as the event or status information, the non-visual interface device 220 generates non-visual information that corresponds to the detected condition information, and outputs the generated information to the user. For example, the non-visual interface device 220 may produce a sound through a speaker or an ear plug of the user to guide the user to move a probe closer to a lesion or produce a vibration when the probe is close to a lesion.

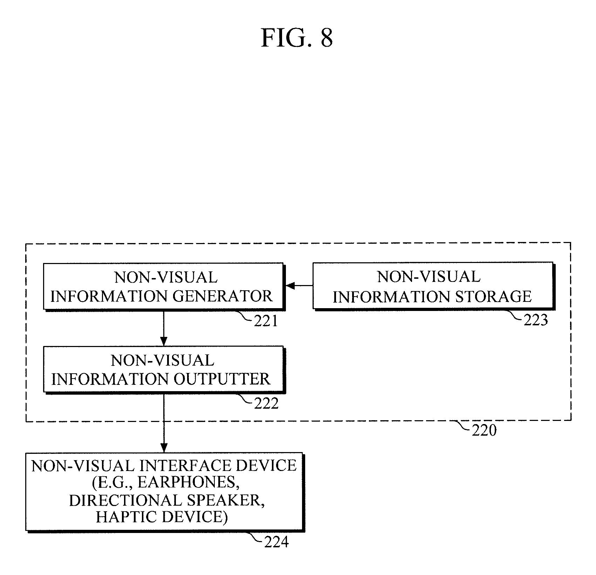

FIG. 8 illustrates in detail an example of the non-visual interface device illustrated in FIG. 6. Referring to FIG. 8, the non-visual interface device 220 includes a non-visual information generator 221, a non-visual information outputter 222, and non-visual information storage 223.

In response to the monitorer 210 detecting the condition information, the non-visual information generator 221 generates non-visual information to be provided to the user by using one or more non-visual forms of expression. Such non-visual forms of expression include, for example, sound, voice, vibration, texture, weight sensation and the like, but not limited thereto.

According to one example, the non-visual information generator 221 determines the forms of non-visual information that are to be provided to the user based on the type of condition information detected by the monitorer 210. The forms of non-visual information that is provided to the user may be defined beforehand based on the types of condition information that are detected, types of non-visual interface device 224 that is connected to the non-visual information providing apparatus 200, or the combination thereof.

In addition, once the form of expression has been determined, the non-visual information generator 221 may determine the output duration, output intensity, number of outputs, or the like, of the non-visual information. Based on these determinations, the non-visual information generator may then generate non-visual information to provide to the user.

For example, in response to a determination by the monitorer 210 that the detected condition information is event information, the non-visual information generator 221 may set the event information to have a sporadic and relatively short output duration (e.g. 1 second) and stronger output intensity than those of status information. Here, the "sporadic and relatively short output duration" may refer to the output occurring at an irregular intervals or in isolation and having an output duration that is equal to or less than a predetermined time period, such as one second. The output duration may be shorter than the average output duration used for indicating status information, for example. In addition, once the monitorer 210 determines that the detected condition information is indeed status information, the non-visual information generator 221 may set the status information to have a longer output duration and a weaker output intensity than those of event information.

In this example, the combination of the type, output duration, intensity and the number of outputs of the non-visual information may be determined based on the type of non-visual interface device 224 that is connected to the non-visual information providing apparatus 200, settings set beforehand, or preferences.

For example, a non-visual interface device, such as a pair of earphones or a directional speaker, may be connected to the non-visual information providing apparatus 200. In response to detecting an activation or a turning-on event of the apparatus, the non-visual interface device may output a continuous high tone beep sound one time for one second. In response to detecting a lesion, the non-visual interface device may output several medium-tone beep sounds or several medium vibrations for one second. The detection of the lesion may be characterized as a lesion detection event. In another example, a verbal, low-tone output such as "lesion detected" may be presented for predetermined number of times for one second.

In this example, in the event that the detected status information indicates a normal input signal, that is, a normal image signal is received after the initial image from the probe, the non-visual information providing apparatus 200 may output a short, high-pitch sound one time in response to an initial image reception event, and thereafter, output a continuous low-tone sound for a predefined period of time in response to the status information. Then, once the lesion detection event is detected and the CAD apparatus enters a lesion tracking status, the non-visual information providing apparatus may adjust the tone or frequency of the sound that is output for the normal input signal status, and may set the sound to be output continuously in proportion to the time spent on tracking the lesion. Then, once status information in relation to determination of the size or severity of the lesion is detected, the non-visual information providing apparatus adjusts a sound volume, a tone and/or a complex sound, and may set the sound to be output continuously in proportion to the determined size or severity of the lesion.

In another example, the non-visual interface device is a haptic device that provides vibrations or weight sensation. The vibration intensity, vibration period, and the number of vibrations provided by the non-visual interface device may be determined depending on the type of detected events or statuses.

In addition, the non-visual information generator 221 may determine whether the currently detected lesion has been previously detected. In the event that it is determined that the current lesion has already been detected in the past, non-visual information generator 221 may not create non-visual information that corresponds to the lesion's detection and tracking status thereof, thus ensuring that the lesion is tracked in silent mode. The non-visual information generator 221 may determine whether a lesion in question has been detected in the past based on any stored image information or annotations regarding the lesion in question. The provision of the non-visual information regarding previously detected lesions may be set by a user in advance.

The non-visual information outputter 222 transmits the generated non-visual information to a non-visual interface device 224, and depending on which form of expression has been chosen, that expression of the non-visual information is provided to the user.

In this event, the non-visual interface device 224 may include, for example, earphones, a haptic device, and a directional speaker that enables sounds to be directed only toward the user; however, the physical structure of the non-visual interface device 224 is not limited thereto. The non-visual interface device 224 may include any interface device capable of providing information in forms that are non-visual. According to one example, a connection between the non-visual interface device 224 and the non-visual information providing apparatus 200 may be established via various types of wired and/or wireless communications, for example, Bluetooth®, WiFi and the like.

According to one example, only the user, for example, a practitioner, who conducts the diagnosis, turns on the non-visual interface device that directs the non-visual information only toward the user by using a directional speaker, so that the non-visual information is relayed only to the user and not to the patient awaiting diagnosis, thereby ensuring that the patient does not get anxious.

The non-visual information storage 223 may store various expressions of non-visual information, such as sound, voice, and vibration, and may be classified and set beforehand by, for example, the status or the type of event, as shown in Table 1 below. However, the aforementioned classification is only provided as an example, and as such the non-visual information may be defined in various ways, and stored in various forms, such as files or databases.

**Table 1**

| Condition Information | Type | Non-visual | Duration | Number of Times | Intensity |
|---|---|---|---|---|---|
| Apparatus Activation | Event | Sound | 1 second | Once | Medium |
| Reception of Initial Image | Event | Sound | 1 second | Once | Strong |
| Normal Input Image | Status | Sound | Continuous | - | Weak |
| Lesion Detected | Event | Sound, Vibration | 1 second | Once | Strong |
| Tracking of Lesion | Status | Vibration | Continuous | - | Weak |
| Assessment of lesion size and severity | Status | Voice, Vibration | Once | Once | Proportional to Size/Severity |

Once the condition information has been detected by the monitorer 210, the non-visual information generator 221 may refer to non-visual information storage 223 to determine which expression of non-visual information corresponds to the detected condition information, the duration in which to output the non-visual information, the number of outputs of the non-visual information, and the intensity at which to output the non-visual information.

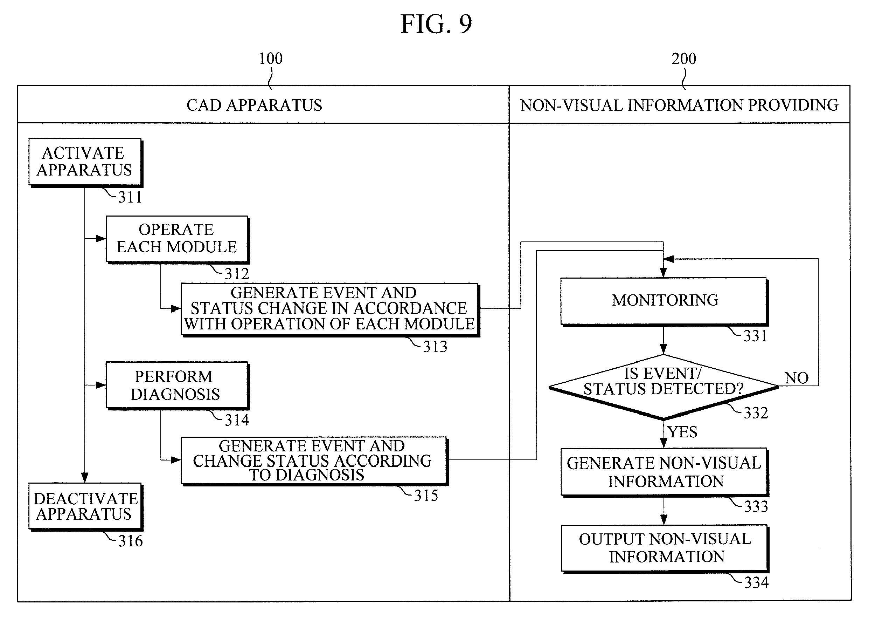

FIG. 9 illustrates a flowchart for an example of a computer-aided diagnosis (CAD) method according to the present disclosure.

The example of the diagnostic method illustrated in FIG. 9 may be performed by the CAD system 1 of FIG. 1.

Referring to FIG. 9, upon activating CAD apparatus 100 through the user's manipulation of, for example, a switch in 311, the CAD apparatus 100 operates each module that is necessary for diagnosis of a patient in 312. In response to the operation of each module, a previously defined event or status change occurs in 313. The CAD apparatus 100 analyzes images that are obtained in accordance with an operation of each module, for example, a probe, and makes a diagnosis based on the analysis in 314, and then generates an event or a status change thereof in accordance with conducting the diagnosis in 315. Operations 312 to 315 are repeatedly performed while images are received, and the apparatus is manually deactivated by the user's manipulation in 316.

The non-visual information providing apparatus 200 monitors the occurrences of events or status changes in the CAD apparatus 100 in 331. In the event that a monitoring result indicates that an event or status information has been detected in 332, the non-visual information providing apparatus 200 generates one or more pieces of non-visual information that corresponds to the detected status information in 333. While a monitoring result indicates that no events or status information has been detected in 332, the non-visual information providing apparatus 200 continues to monitor the CAD apparatus 100 in 331. The non-visual information that is generated in response to the detection of an event or a status change is transmitted to a connected non-visual interface device, such as a haptic device and a directional speaker, allowing for the non-visual information to be provided to the user in 334.

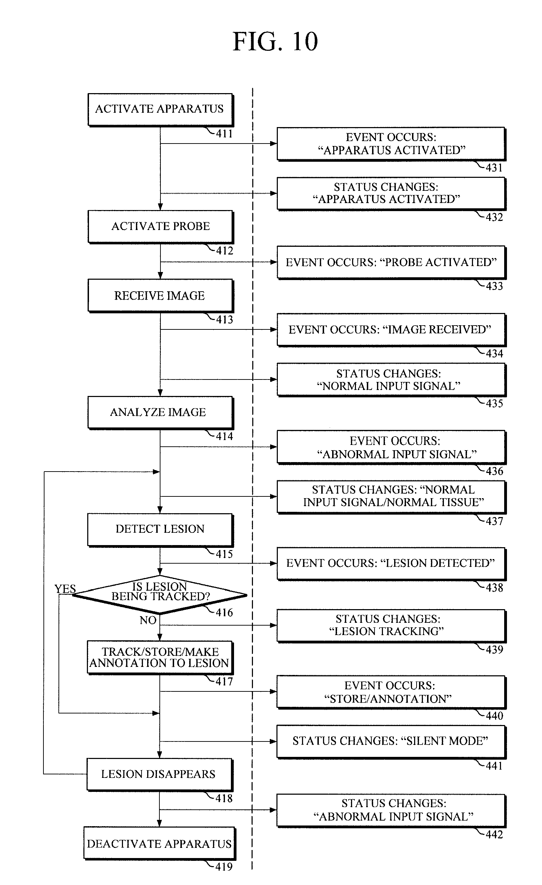

FIG. 10 illustrates a flowchart for a CAD method performed by the CAD apparatus of FIG. 9 to facilitate an ultrasonic diagnosis of a patient.

Referring to FIG. 10, when a user activates the CAD apparatus to diagnose the patient in 411, the CAD apparatus creates an apparatus activation event to indicate that the apparatus is working normally in 431. In 432, the CAD apparatus changes its status to "apparatus activated."

In response to a user activating a probe to obtain ultrasonic images from the patient in 412, the CAD apparatus creates "probe activated" event to indicate that the probe is normally operated in 433.

After having received images sequentially from the probe that is put in close contact with the affected part of the patient, the CAD apparatus creates an "image received" event to indicate that an initial image has been received in 434. Thereafter, the CAD apparatus changes its status to "input signal normal" status to indicate that images are being normally received in 435.

Then, in 414, the images from the probe are analyzed automatically or manually in response to the user's input, and diagnosis is made based on the analysis. If the user moves the probe at a speed greater than a predetermined speed or beyond the affected part, so that it is impossible for the CAD apparatus to receive images normally, the CAD apparatus creates an "abnormal input signal" event to inform the user in 436. In addition, if images are continuously received and no lesions are detected from the received images, the CAD apparatus changes its status to "normal input signal/normal tissue" status in 437. The non-visual information providing apparatus continuously outputs to the user non-visual information that corresponds to the current status, so that the user does not need to concentrate on the screen while the same status continues, thereby alleviating the user's effort during diagnosis and increasing the speed of manipulating the probe, thus allowing for quick diagnosis.

Then, in response to detecting a lesion thorough image analysis in 415, the CAD apparatus creates a "lesion detected" event in 438 to inform the user that a lesion has been detected. In 416, it is determined whether the lesion in question has been previously detected and is thus currently being tracked. If it is determined that the current lesion has been detected before, the status is changed to a "lesion tracking" event in 439 to inform the user that the lesion in question is being tracked, as shown in 417. At this time, in the event that the user recognizes the detected lesion and stores the image that includes the detected lesion or makes annotations regarding the detected lesion in 417, the CAD apparatus creates a "store/annotation" event in 440 to indicate that a lesion image is stored or an annotation is made to the lesion in question, and may automatically changes its status to silent mode in 441 in order not to provide additional information to the user any longer since the user is already aware of the lesion.

In the event that it is determined, as in 416, that the currently detected lesion has been detected before and tracking of the lesion is ongoing or completed, the CAD apparatus automatically changes its status to silent mode in 441 so as not to provide the user with additional information about the lesion.

Thereafter, in the event that no lesions appear any longer in the received image in 418 and the CAD apparatus continues to receive an image, operations subsequent to operation 414, in which the image is analyzed, are repeatedly performed. When the user completes diagnosing a patient and removes the probe from the affected part of the patient, the CAD apparatus changes its status to abnormal-input-signal status in 442, and the user deactivates the CAD apparatus in 419.

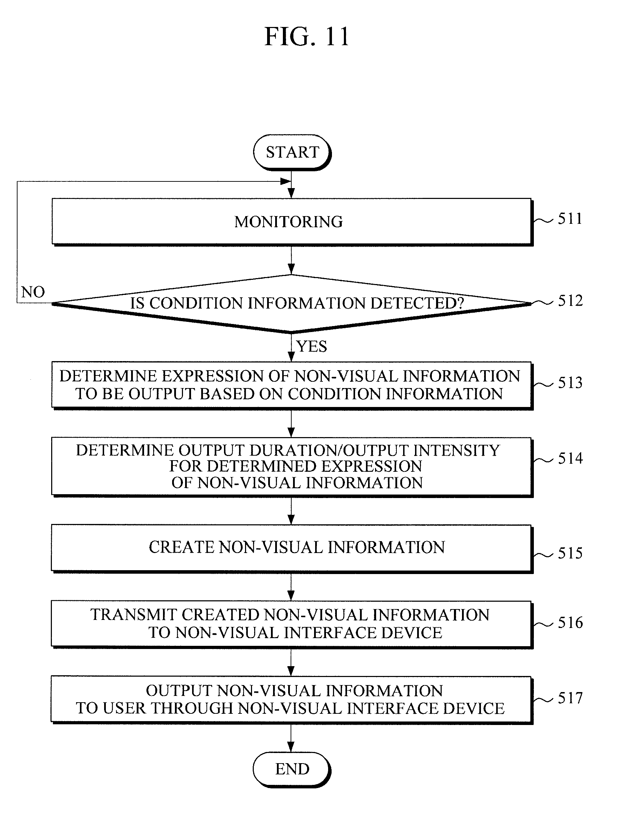

FIG. 11 illustrates a flowchart for an example of a CAD method performed by the non-visual information providing apparatus of FIG. 9.

Referring to FIG. 11, in 511, the non-visual information providing apparatus constantly monitors an event or status change that occurs in the CAD apparatus, as shown in FIG. 10. The monitoring of the CAD apparatus is consistently performed until condition information is detected in the CAD apparatus or the CAD apparatus is deactivated.

In the event that condition information is detected in 512 and the condition information relates to, for example, the occurrence of an event or status change is detected in the CAD apparatus in 512, the non-visual information providing apparatus determines an expression of non-visual information to be provided to the user based on the detected condition information in 513. For example, one or more expressions of non-visual information such as voice, sound, vibration, weight sensation, and the like may be chosen based on the type of non-visual interface device or the type of detected condition information. In addition, a preferred expression of non-visual information for each user may be designated beforehand based on user characteristics or pre-designated user choice.

In response to the determination of the expression of non-visual information, output duration, the number of outputs, and output intensity are determined for each determined expression in 514. The output duration, the number of outputs and output intensity may vary depending on the type of condition information, that is, event information or status information, and the user characteristics.

Then, in 515, non-visual information to be output to the user is created using the expression of non-visual information, the output duration, the number of outputs, and the output intensity, which are determined for the detected condition information.

Thereafter, in 516, the created non-visual information is transmitted to the connected non-visual interface device, and then provided to the user through the non-visual interface device in 517.

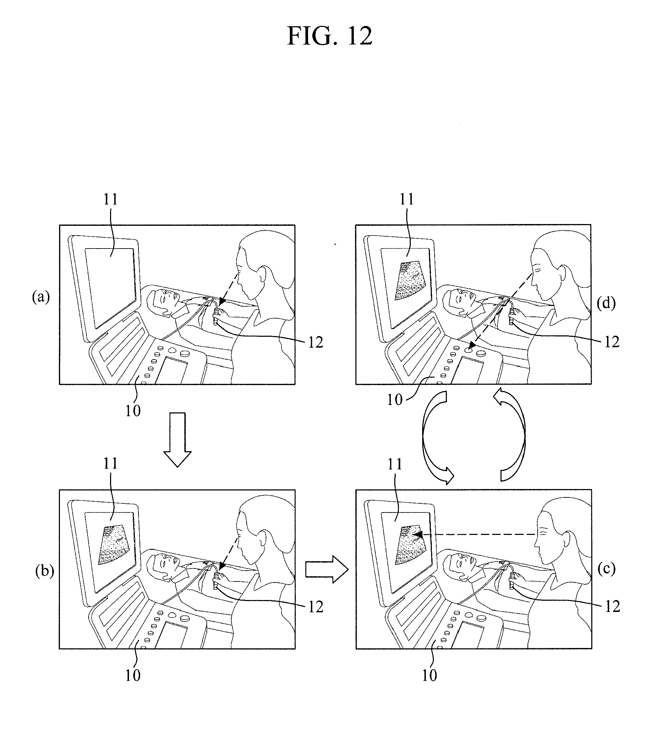

FIG. 12 illustrates an example of the application of a general CAD system.

Referring to FIG. 12, (a) a user activates a general CAD system 10 and places a probe in close contact with an affected part of a patient while watching the patient; (b) an image obtained by the probe is output to a screen 11; (c) the user checks the image and diagnostic information while viewing the screen 11; and (d) in the event that a lesion or the like is found, the user additionally manipulates the CAD system, or otherwise, turns his/her eyes to the patient and moves the probe 12. As such, the user who uses the general CAD system may frequently switch or divide his/her attention between the system and the patient in order to check the system equipment and the patient while operating the equipment to save or input relevant information. By doing so, the user risks missing a significant lesion from the image while the user takes his/her eyes off from the screen.

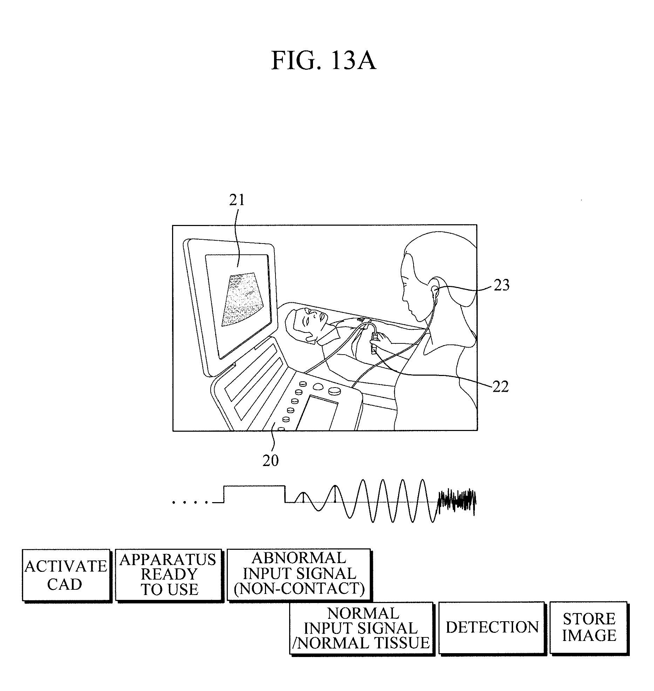

FIG. 13A illustrates an example of the application of a CAD system in accordance with the present disclosure.

Referring to FIG. 13A, a user performs a diagnosis with a non-visual audio interface device 23 in ears, wherein the non-visual audio interface device is capable of sound and voice transmission and is connected to a CAD system 20. In response to the user acquiring an image from the CAD system 20 and making a diagnosis based on the obtained image, the CAD system creates a predefined event for each diagnostic procedure, or changes its status, and a non-visual information providing apparatus in accordance with the illustrated example monitors the created event or the status change of the CAD apparatus. In response to detection of the event or status change, the non-visual information providing apparatus checks the connected non-visual audio interface device, and generates appropriate non-visual information to be output to the non-visual interface device. That is, since the user is putting on earphones as shown in FIG. 13A, the non-visual information providing apparatus detects the connected earphones, creates non-visual information in the form of sound or voice, and transmits the created information to the earphones. Although the earphones in this case are connected to the CAD apparatus via a wire as illustrated, it may not be limited to the wire connection and may be connected via wireless communication, such as Bluetooth®. The non-visual information providing apparatus transmits the created non-visual information according to a communication method of the connected earphones.

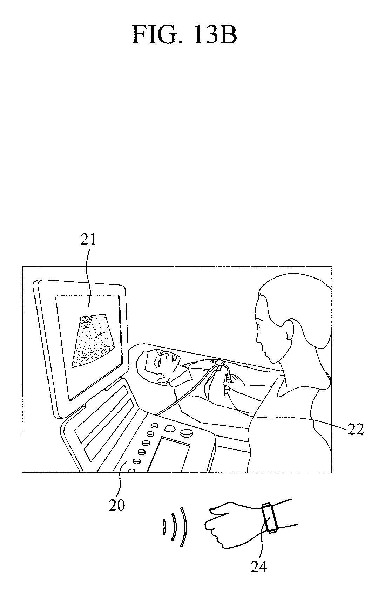

FIG. 13B illustrates another example of the application of a CAD system.

In the example illustrated in FIG. 13B, a user wears a haptic device 24 on his/her wrist, and the haptic device 24 creates vibrations or weight sensation to guide the user in moving the probe around a patient. To guide the user, an event created by a CAD apparatus or status change of the CAD apparatus is detected by a non-visual information providing apparatus, and the appropriate non-visual information is created in accordance with the capability of the haptic device 24. For example, the non-visual information provided to the user may be vibration or weight sensation, and the created non-visual information may be transmitted via the haptic device 24 so as to be output to the user.

Accordingly, the CAD system 20 provides relevant non-visual information to the user through the earphones 23 or the haptic device 24 in the process of examining the patient by obtaining the images through various operations or the probe 22, so that it is possible to minimize frequent switching or dividing of user's attention among the screen 21, the patient, and other places of the CAD system 20. With this example, in the event that no lesion is detected in ultrasound images that are received in real time, the user can observe the patient for a predetermined period of time, and then switch his/her eyes to the screen 21 to examine the lesion more closely.

The methods and/or operations described above may be recorded, stored, or fixed in one or more computer-readable storage media that includes program instructions to be implemented by a computer to cause a processor to execute or perform the program instructions. The media may also include, alone or in combination with the program instructions, data files, data structures, and the like. Examples of computer-readable storage media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The described hardware devices may be configured to act as one or more software modules in order to perform the operations and methods described above, or vice versa. In addition, a computer-readable storage medium may be distributed among computer systems connected through a network and computer-readable codes or program instructions may be stored and executed in a decentralized manner.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A non-visual information providing apparatus comprising:
a monitorer configured to detect condition information generated by a computer-aided diagnosis (CAD) apparatus;
a non-visual information generator configured to generate non-visual information based on the detected condition information; and
a non-visual information outputter configured to output the generated non-visual information to a user.

2. The apparatus of claim 1, wherein the non-visual information generator is configured to select at least one expression of non-visual information from sound, voice or vibration, determine output duration and output intensity for the determined expression of non-visual information, and generate the non-visual information.

3. The apparatus of claim 2, wherein the condition information comprises status information regarding a status of the CAD apparatus or event information regarding an event that occurred in the CAD apparatus.

4. The apparatus of claim 3, wherein the non-visual information generator is configured to, in response to the detected condition information being event information, set the output duration to be equal to or less than a predetermined length of time and the output intensity to be equal to or greater than a predetermined intensity for the determined expression of non-visual information, and in response to the detected condition information being status information, set the output duration to be longer than the predetermined length of time and the output intensity to be weaker than the predetermined intensity of the event information.

5. The apparatus of claim 3, wherein the event information comprises information generated due to an activation or deactivation of the CAD apparatus, a status change of the CAD apparatus, a detection of a lesion, a reception of an image, a speed of moving probe, an operational range of the probe, or a combination thereof.

6. The apparatus of claim 5, wherein the non-visual information generator is configured to, in response to the detected condition information relating to detection of a lesion, determine whether the detected lesion has been previously detected, and in response to a determination that the lesion has been detected before, generate neither an event relating to the detected lesion nor non-visual information relating to a status.

7. The apparatus of claim 3, wherein the status information comprises information generated due to a tracking of a lesion, a size of the lesion, a severity of a lesion, a probe position or a combination thereof.

8. The apparatus of claim 7, wherein, in response to the detected condition information relating to a tracking of a lesion, the non-visual information generator is configured to set the output duration and the output intensity in proportion to a time spent on tracking a lesion.

9. The apparatus of claim 7, wherein, in response to the detected condition information relating to a size or a severity of a lesion, the non-visual information generator is configured to set the output duration and the output intensity in proportion to a size or severity of a lesion.

10. The apparatus of claim 1, wherein the non-visual information outputter is configured to output the generated non-visual information to a user through a non-visual interface device;
the non-visual interface device is connected to the non-visual information outputter via a wire or wireless communication; and
the non-visual interface device comprises one or more of a headset, an earphone, a directional speaker, and a haptic device.

11. A method of providing non-visual information, the method comprising:
detecting condition information generated in a computer-aided diagnosis (CAD) apparatus;
generating non-visual information based on the detected condition information; and
outputting the generated non-visual information to a user through a non-visual interface device.

12. The method of claim 11, wherein the generating of the non-visual information comprises:
determining at least one expression of non-visual information from sound, voice, and vibration, based on the detected condition information;
determining output duration and output intensity for the determined expression of non-visual information, based on the detected condition information; and
generating the non-visual information using the determined output duration and output intensity.

13. The method of claim 12, wherein the determining of the output duration and output intensity comprises:
in response to the detected condition information being event information, setting the output duration to be equal to or less than a predetermined length of time and the output intensity to be equal to or greater than a predetermined intensity for the determined expression of non-visual information; and
in response to the detected condition information being status information, setting the output duration to be longer than the predetermined length of time and the output intensity to be weaker than the predetermined intensity.

14. The method of claim 12, wherein, in response to the detected condition information relating to tracking of lesion, the determining of the output duration and output intensity comprises setting the output duration and the output intensity in proportion to a time spent on tracking a lesion.

15. The method of claim 12, wherein, in response to the detected condition information relating to the size or severity of a lesion, the determining of the output duration and output intensity comprises setting the output duration and the output intensity in proportion to a size or severity of a lesion.
